# EUROPÄISCHE PATENTANMELDUNG

(11) **EP 0 998 933 A1**
(43) Veröffentlichungstag der Anmeldung: **10.05.2000**
(21) Anmeldenummer: 98119103.4
(22) Anmeldetag: 09.10.1998
(51) Int. Cl.: A61K 31/66, A61K 9/20

(54) **Verfahren zur Herstellung von bisphosphonathaltigen pharmazeutischen Zusammensetzungen zur oralen Applikation**

(71) Anmelder: Roche Diagnostics GmbH, 68305 Mannheim (DE)
(72) Erfinder: Möckel, Jörn, 69221 Dossenheim (DE); Gabel, Rolf-Dieter, Dr., 68723 Schwetzingen (DE); Woog, Heinrich, Dr., 69514 Laudenbach (DE)

(57) **Zusammenfassung**

Die Erfindung betrifft ein Verfahren zur Herstellung von bisphosphonathaltigen pharmazeutischen Zusammensetzungen zur oralen Applikation mit einem niedrigen Wirkstoffgehalt ≤ 50 mg pro Darreichungsform, das dadurch gekennzeichnet ist, daß man den Bisphosphonat-Wirkstoff in an sich bekannter Weise in einem Wirbelschichtgranulator feucht granuliert, wobei Hilfsstoffe verwendet werden, die keine abrasive Wirkung zeigen, das feuchte Granulat anschließend in der Wirbelschicht trocknet, über ein Sieb passender Maschenweite siebt und nach an sich bekannten Techniken zu pharmazeutischen Zusammensetzungen weiterverarbeitet, wobei der Mindergehalt des Wirkstoffs im Vergleich zur Ausgangswirkstoffmenge auf weniger als 6 Gew.-% reduziert wird.

## Beschreibung

Die Erfindung betrifft ein Verfahren zur Herstellung von pharmazeutischen Zusammensetzungen von Aminoalkyl-1,1-diphosphonsäure-Derivaten oder deren physiologisch unbedenklichen Salzen (im folgenden allgemein Bisphosphonate genannt) zur oralen Applikation mit einem niedrigen Wirkstoffgehalt ≤ 50 mg pro Darreichungsform. Durch das Verfahren wurde der Mindergehalt des Bisphosphonat-Wirkstoffs im Vergleich zur eingesetzten Menge auf weniger als 6 Gew.-% reduziert.

Bisphosphonate sind von Interesse bei der Behandlung von Knochenerkrankungen und bestimmten Störungen des Calciumhaushalts, wie z.B. der Hyperkalzämie, Osteoporose, Tumorosteolyse, Morbus Paget, u. a..

An pharmazeutische Zubereitungen sind allgemein hohe Ansprüche hinsichtlich Gehalt, Gehaltseinheitlichkeit und Reinheit zu stellen. Spezielle Wirkstoffeigenschaften können Gehalt, Gehaltseinheitlichkeit und Reinheit der Darreichungsform negativ beeinflussen. Es ist bekannt, daß Bisphosphonate eine Substanzgruppe darstellen, die stark zur Komplexierung mehrwertiger Metallionen neigt. Da bei Herstellung üblicher pharmazeutischer Zubereitungen zur oralen Applikation gewöhnlich Anlagen und Geräte mit metallischen Oberflächen zum Einsatz kommen, kommt es bei Verarbeitung von Bisphosphonaten zum Kontakt des stark komplexierenden Wirkstoffes mit komplexierbarem Material. Dies ist insbesondere dann der Fall, wenn die Verarbeitung unter Einsatz von Wasser oder wäßrigen Medien erfolgt. Abhilfe schafft die trockene Verarbeitung, speziell nach dem Prinzip der Direkttablettierung, da hier die Feuchtgranulation vermieden wird. Die Direkttablettierung ist ein Verfahren, das zur Herstellung hoch dosierter Tabletten gut geeignet ist. Bekanntermaßen sind jedoch insbesondere hochdosierte orale Darreichungsformen von Bisphosphonaten mit Verträglichkeitsproblemen behaftet, was die orale Behandlug erschwert. So führen besonders Aminodiphosphonsäuren zu Irritationen des oberen Gastrointestinaltraktes (Fleisch H, Bisphosphonates in Bone Disease, Herben Fleisch, Bern 1993; S. 126-131). Weiterhin besteht bei der Direkttablettierung, wie auch bei der Abfüllung nicht granulierter Pulvermassen in Gelatinekapseln, das Risiko von Gehaltsschwankungen, speziell bei niedrig oder sehr niedrig dosierten Wirkstoffen. Aus diesem Grunde kann auf die Feuchtgranulation trotz der genannten Komplexierungsgefahr nicht verzichtet werden. Bei Einsatz von Schnellmischern wird der Wirkstoff mit Hilfsstoffen gemischt und mit Wasser oder wäßriger Bindemittellösung feucht granuliert. Dabei wird der Wirkstoff auf äußerst intensive Weise mit der metallischen Geräteoberfläche in Kontakt gebracht. Die Gefahr der Komplexbildung kann durch die abrasive Wirkung bestimmter pharmazeutischer Hilfsstoffe noch zusätzlich verstärkt werden.

Die Aufgabe der Erfindung bestand deshalb darin, ein Herstellungsverfahren für niedrig dosierte bisphosphonathaltige pharmazeutische Zusammensetzungen zur oralen Applikation mit einem Gehalt ≤ 50 mg Wirkstoff pro Darreichungsform zu entwickeln, mit dem der Verlust an Wirkstoff bei der Herstellung der Zusammensetzungen im Vergleich zur Ausgangswirkstoffmenge gesenkt werden kann.

Die Aufgabe wird dadurch gelöst, daß Bisphosphonate unter Verwendung der an sich bekannten Wirbelschichtgranulation in oral applizierbare Formulierungen, die den Wirkstoff in einer niedrigen Dosierung ≤ 50mg pro Darreichungsform enthalten, überführt werden. Bei der Wirbelschichtgranulation handelt es sich ebenfalls um ein klassisches Feuchtgranulationsverfahren. Überraschend kann jedoch mit diesem Verfahren der Mindergehalt des Wirkstoffs im Vergleich zur eingesetzten Menge Wirkstoff auf weniger als 6 Gew.-%, bevorzugt weniger als 4 Gew.-% gesenkt werden.

Der erwähnte Nachteil einer Komplexierung bei Feuchtgranulierungen, stellt für die Herstellung niedrig dosierter Bisphosphonat-Wirkstoffe somit kein Problem mehr dar. Bevorzugt werden pharmazeutische Zusammensetzungen mit einem Gehalt ≤ 50 mg, insbesondere ≤ 10 mg Bisphosphonat, pro Darreichungsform hergestellt. Die Herstellung der erfindungsgemäßen pharmazeutischen Zusammensetzungen erfolgt durch die Granulierung des Wirkstoffs in einem an sich bekannten Wirbelschichtgranulator unter Verwendung von Hilfsstoffen, die bei der Verarbeitung in pharmazeutisch üblichen Produktionsanlagen keine abrasive Wirkung zeigen, wie dies z.B. bei Siliciumdioxid der Fall ist.

Vorzugsweise wird der Wirkstoff als Lösung oder Suspension zusammen mit einer wäßrigen Bindemittellösung auf geeignete andere Hilfsstoffe aufgesprüht und granuliert oder der Wirkstoff wird mit den Hilfsstoffen als trockenes Pulver im Wirbelschichtgranulator vorgelegt und die Granulation erfolgt durch Einsprühen von Wasser in die Pulvermischung, wobei ein Bindemittel hier in der Pulvermischung enthalten ist.

Das erhaltene feuchte Granulat wird anschließend im Wirbelschichtgranulator getrocknet, bis eine für die Weiterverarbeitung zur pharmazeutischen Zusammensetzung mit anderen Maschinen geeignete Masse mit akzeptabler Restfeuchte vorliegt. Das getrocknete Granulat wird über ein Sieb passender Maschenweite gegeben und danach mit an sich bekannten Techniken, ggf. unter Zumischung weiterer Hilfsstoffe, weiterverarbeitet.

Erfindungsgemäß verwendete Wirkstoffe sind die Bisphosphonate: (4-Amino-1-hydroxybutyliden)bis-phosphonat (Alendronat), (Dichlormethylen)bis-phosphonat (Clodronat), [1-Hydroxy-3-(1-pyrrolidinyl)-propyliden]bis-phosphonat (EB-1053), (1-Hydroxyethyliden)bis-phosphonat (Etidronat), [1-Hydroxy-3-(methylpentylamino)propyliden]bis-phosphonat (Ibandronat), [Cycloheptylamino)-methylen]bis-phosphonat (Incadronat), (6-Amino-1-hydroxyhexyliden)bis-phosphonat (Neridronat), [3-(Dimethylamino)-1-hydroxypropyliden]bis-phosphonat (Olpadronat), (3-Amino-1-hydroxypropyliden)bis-phosphonat (Pamidronat), [1-Hydroxy-2-(3-pyridinyl)ethylenl]bis-phosphonat (Risedronat), [[(4-Chlorphenyl)thiol]-methylen]bis-phosphonat (Tiludronat), [1-Hydroxy-2-imidazo-(1,2-a)pyridin-3-ylehtyliden]bis-phosphonat (YH 529), [1-Hydroxy-2-(1H-imidazol-1-yl)ethyliden]bis-phosphonat (Zoledronat), die in Form der freien Säuren oder als pharmazeutisch verträgliche Salze, insbesondere als Natriumsalze eingesetzt werden.

Gemäß der Erfindung werden bevorzugt pharmazeutische Zubereitungen hergestellt, die den Wirkstoff in einer Menge von ≤ 50 mg pro Darreichungsform enthalten, vorzugsweise ≤ 10 mg, besonders bevorzugt 0,1 bis 5 mg und 0,1 bis 2,5 mg. Als besonders bevorzugter Wirkstoff wird Ibandronat eingesetzt.

Als Hilfsstoffe, die bei der Granulation keine abrasive Wirkung zeigen, werden zur Herstellung der pharmazeutischen Zusammensetzungen Verbindungen eingesetzt, die ausgewählt werden aus Füllmitteln, wie z.B. Lactose in seiner Form als Hydrat oder Anhydrat, Zuckeralkoholen, wie z.B. Mannit, Tablettierhilfsstoffen, wie z.B. Cellulose in mikrokristalliner oder fasriger Form, und Bindemitteln, wie z.B. Polyvinylpyrrolidon oder Celluloseethern, wie z.B. Methylhydroxypropylcellulose. Dabei wird mindestens ein Hilfsstoff eingesetzt, der als Bindemittel fungiert.

Vorzugsweise wird mindestens einer der Hilfsstoffe Lactose, mikrokristalline Cellulose, und Polyvinylpyrrolidon verwendet. In einer bevorzugten Ausführungsform werden 1-99% Lactose, 1-99% mikrokristalline Cellulose, 0,1-20% Polyvinylpyrrolidon und 0-5% Stearinsäure eingesetzt, besonders bevorzugt 25-75% Lactose, 10-20% mikrokristalline Cellulose, 2-3% Polyvinylpyrrolidon und 0-3% Stearinsäure, ganz besonders bevorzugt 0,05-3% Stearinsäure.

Die Weiterverarbeitung des Granulates erfolgt nach an sich bekannten Verfahren, ggf. unter Zusatz weiterer Hilfsstoffe, zu Tabletten, Kautabletten, Brausetabletten, Filmtabletten, zu Dragees und Pellets sowie durch Abfüllung in Hartgelatinekapseln oder Sachets. Hilfsstoffe für die Weiterverarbeitung sind an sich übliche Schmiermittel, Zerfallshilfsstoffe, Fließregulierungsmittel, Tablettierungshilfsmittel u.a. Durch die erfindungsgemäße Herstellung der pharmazeutischen Zusammensetzungen unter Einsatz der Wirbelschichtgranulation, insbesondere durch den Trocknungsvorgang im Wirbelschichtgranulator, kommt es zu einem weniger intensiven Kontakt des Materials mit der Anlagenoberfläche, wodurch es zu der überraschend erheblichen Reduzierung des Mindergehaltes der Wirkstoffe kommt, was insbesondere bei den niedrigen Dosierungen von großem Vorteil ist. Somit können die oben beschriebenen Nachteile, die bei der bisher üblichen Herstellung von geeigneten oralen Darreichungsformen auftreten, weitgehend vermieden werden.

Nachfolgend soll die Erfindung an Beispielen näher erläutert werden, ohne sie darauf zu beschränken.

### Beispiel 1 (Vergleichsbeispiel):

### Mindergehalt bei Herstellung von Ibandronat 2,5 mg-Kapseln nach Granulation im Schnellmischer/Granulator

Lactose, Ibandronat und Polyvinylpyrrolidon werden im Schnellmischer/Granulator (Typ Diosna) bei einem Füllungsgrad von 50 % 2 Minuten gemischt und anschließend 8 Minuten mit Wasser granuliert. Das feuchte Granulat wird in der Wirbelschicht getrocknet (Gerätetyp Aeromatic), über ein 0,8 mm Sieb gesiebt, mit Zerfallshilfsstoff und Schmiermittel gemischt (Mischertyp Rhoenrad, Mischzeit 10 Minuten) und auf einer Kapselmaschine (Typ MG2/G36) bei einer Maschinenleistung von 20 000 St/Stunde ohne Komprimierung in Hartgelatinekapseln der Größe 2 verkapselt.

| | |
|---|---|
| Sollgewicht der Füllmasse: | 200,0 mg |
| Istgewicht der Füllmasse lt. Inprozeßkontrolle: | 200,9 mg |
| Wirkstoffgehalt der Kapseln: | 94,8 % ± 5,2 %, (n=10 Einzelmessungen) |

### Beispiel 2 (Vergleichsbeispiel):

### Mindergehalt bei Herstellung von Ibandronat 1,0 mg-Kapseln nach Granulation im Schnellmischer/Granulator

Lactose, Ibandronat und Polyvinylpyrrolidon werden im Schnellmischer/Granulator (Typ Diosna) 2 Minuten gemischt und anschließend 10 Minuten mit Wasser granuliert. Das feuchte Granulat wird in der Wirbelschicht getrocknet (Gerätetyp Aeromatic), über ein 0,8 mm Sieb gesiebt, mit Zerfallshilfsstoff und Schmiermittel gemischt (Mischertyp Rhoenrad, Mischzeit 10 Minuten) und auf einer Kapselmaschine (Typ KFM Harro Höfliger) in Hartgelatinekapseln der Größe 2 verkapselt.

| | |
|---|---|
| Sollgewicht der Füllmasse: | 220,00 mg |
| Istgewicht der Füllmasse lt. Inprozeßkontrolle: | 220,05 mg |
| Wirkstoffgehalt der Kapseln: | 94,9 % ± 1,9 %, (n=10 Einzelmessungen) |

### Beispiel 3:

### Gehalt innerhalb der Akzeptanzgrenzen bei Herstellung von Ibandronat 1,0 mg-Tabletten nach Granulation in der Wirbelschicht

Lactose und mikrokristalline Cellulose werden im Wirbelschichtgranulator (Typ Aeromatic) mit einer wäßrigen Lösung von Polyvinylpyrrolidon und Ibandronat granuliert. Das feuchte Granulat wird in der Wirbelschicht getrocknet (Typ Aeromatic), über ein 1,0 mm Sieb gesiebt, mit Zerfallshilfsstoff, Schmiermittel, Fließregulierungsmittel und mikrokristalliner Cellulose gemischt (Mischertyp Turbula, Mischzeit 5 Minuten) und auf einer Tablettenpresse (Typ Korsch) bei einer Maschinenleistung von 25 000 St/Stunde zu Tabletten verpreßt.

| | |
|---|---|
| Sollgewicht der Tabletten: | 100,0 mg |
| Istgewicht der Tabletten lt. Inprozeßkontrolle: | 101,3 mg |
| Wirkstoffgehalt der Tabletten: | 98,3 % ± 4,2 %, (n=10 Einzelmessungen) |

### Beispiel 4:

### Gehalt innerhalb der Akzeptanzgrenzen bei Herstellung von Ibandronat 0,1 mg-Tabletten nach Granulation in der Wirbeischicht

Lactose und mikrokristalline Cellulose werden im Wirbelschichtgranulator (Typ Aeromatic) mit einer wäßrigen Lösung von Polyvinylpyrrolidon und Ibandronat granuliert. Das feuchte Granulat wird in der Wirbeischicht getrocknet (Typ Aeromatic), über ein 1,0 mm Sieb gesiebt, mit Zerfallshilfsstoff, Schmiermittel, Fließregulierungsmittel und mikrokristalliner Cellulose gemischt (Mischertyp Turbula, Mischzeit 10 Minuten) und auf einer Tablettenpresse (Typ Korseh) bei einer Maschinenleistung von 60 000St/Stunde zu Tabletten verpreßt.

| | |
|---|---|
| Sollgewicht der Tabletten: | 100,0 mg |
| Istgewicht der Tabletten lt. Inprozeßkontrolle: | 101,3 mg |
| Wirkstoffgehalt der Tabletten: | 98,5 % ± 2,4 % (n=10 Einzelmessungen) |

## Patentansprüche

1. Verfahren zur Herstellung von bisphosphonathaltigen pharmazeutischen Zusammensetzungen zur oralen Applikation mit einem niedrigen Wirkstoffgehalt ≤ 50 mg pro Darreichungsform, dadurch gekennzeichnet, daß man den Bisphosphonat-Wirkstoff in an sich bekannter Weise in einem Wirbelschichtgranulator feucht granuliert, wobei Hilfsstoffe verwendet werden, die keine abrasive Wirkung zeigen, das feuchte Granulat anschließend in der Wirbeischicht trocknet, über ein Sieb passender Maschenweite siebt und nach an sich bekannten Techniken zu pharmazeutischen Zusammensetzungen weiterverarbeitet, wobei der Mindergehalt des Wirkstoffs im Vergleich zur Ausgangswirkstoffmenge auf weniger als 6 Gew.-% reduziert wird.

2. Verfahren nach Anspruch 1, dadurch gekennzeichnet, daß man den Bisphosphonat-Wirkstoff als Lösung oder Suspension zusammen mit einem wäßrigen Bindemittel auf andere Hilfsstoffe aufsprüht und granuliert.

3. Verfahren nach Anspruch 1, dadurch gekennzeichnet, daß man den Bisphosphonat-Wirkstoff mit den Hilfsstoffen als trockenes Pulver im Wirbelschichtgranulator vorlegt und ihn durch Einsprühen von Wasser in die Pulvermischung granuliert, wobei ein Bindemittel in der Pulvermischung enthalten ist.

4. Verfahren nach einem der Ansprüche 1 bis 3, dadurch gekennzeichnet, daß man als Bisphosphonat Alendronat, Clodronat, EB-1053, Etidronat, Ibandronat, Incadronat, Neridronat, Olpadronat, Pamidronat, Risedronat, Tiludronat, YH 529, Zoledronat in Form der freien Säure oder als pharmazeutisch verträgliches Salz, insbesondere als Natriumsalz, einsetzt.

5. Verfahren nach Anspruch 4, dadurch gekennzeichnet, daß man als Wirkstoff Ibandronat einsetzt.

6. Verfahren nach einem der Ansprüche 1 bis 5, dadurch gekennzeichnet, daß man mindestens einen der Hilfsstoffe Lactose, mikrokristalline Cellulose oder Polyvinylpyrrolidon verwendet.

7. Verfahren nach Anspruch 6, dadurch gekennzeichnet, daß man 1-99% Lactose, 1-99% mikrokristalline Cellulose, 0,1-20% Polyvinylpyrrolidon und 0-5% Stearinsäure verwendet.

8. Verfahren nach Anspruch 7, dadurch gekennzeichnet, daß man 25-75% Lactose, 10-20% mikrokristalline Cellulose, 2-3% Polyvinylpyrrolidon, 0-3% Stearinsäure besonders bevorzugt 0,05-3% Stearinsäure, verwendet.

9. Verfahren nach einem der Ansprüche 1 bis 8, dadurch gekennzeichnet, daß die Weiterverarbeitung zu Tabletten, Kapseln, Filmtabletten, Dragees, Pellets, Brausetabletten, Kautabletten oder Granulaten in Sachets erfolgt.

10. Verfahren nach Anspruch 9, dadurch gekennzeichnet, daß die Weiterverabeitung unter Zumischung weiterer Hilfsstoffe erfolgt.

11. Verfahren zur Reduzierung des Mindergehaltes von Bisphosphonat-Wirkstoffen in niedrig dosierten Darreichungsformen für die orale Applikation auf weniger als 6 Gew.-%, dadurch gekennzeichnet, daß die Darreichungsformen hergestellt werden, indem man den Bisphosphonat-Wirkstoff in an sich bekannter Weise in einem Wirbelschichtgranulator feucht granuliert, wobei Hilfsstoffe verwendet werden, die keine abrasive Wirkung zeigen, das feuchte Granulat anschließend in der Wirbeischicht trocknet, über ein Sieb passender Maschenweite siebt und nach an sich bekannten Techniken zu pharmazeutischen Zusammensetzungen weiterverarbeitet.
